# EUROPEAN PATENT SPECIFICATION

(11) **EP 1 811 302 B1**
(45) Date of publication and mention of the grant of the patent: **10.08.2011**
(21) Application number: 06090012.3
(22) Date of filing: 19.01.2006
(51) Int. Cl.: G01N 33/543, G01N 21/55

(54) **Diagnostic nanosensor and its use in medicine**
Diagnostischer Nanosensor und seine Verwendung
Nano-capteur diagnostique et son usage

(43) Date of publication of application: 25.07.2007
(73) Proprietor: GILUPI GmbH, 12487 Berlin (DE)
(72) Inventor: Pison, Ulrich, 14163 Berlin (DE); Giersig, Michael, 53175 Bonn (DE); Schäfer, Axel, 10713 Berlin (DE)
(74) Representative: Grünecker, Kinkeldey, Stockmair & Schwanhäusser Anwaltssozietät

(56) References cited:
- WO-A-2004/086044
- WO-A-2005/031299
- WO-A-2005/093831
- FR-A- 2 860 872
- US-A1- 2002 111 551
- US-A1- 2005 142 030
- US-A1- 2005 157 445

## Description

### FIELD OF THE INVENTION

This invention relates generally to biosensor technology, and pertains more particularly to novel multifunctional biosensors based on ordered arrays of metallic, semiconductors and magnetic nano-islands for medical, biological, biochemical, chemical and environmental applications.

Specifically, the present invention relates to a Diagnostic Nanosensor according to claim 1.

FR 2 860 872 A discloses a Nanodetector comprising an array of spots made from an electrically conductive material on a substrate and capable of immobilizing chemical or biological targets, the spots having dimensions of less than 1µm. The spots have circular or elliptical shapes.

### BACKGROUND OF THE INVENTION

The nanoscale science and engineering have shown great promise for the fabrication of novel biosensors with faster response and higher sensitivity than that of planar sensor configurations, due to their small dimensions combined with a dramatically increased contact surface and strong binding with biological and chemical reagents. Such biosensors have important applications in medicine, in biological and biochemical research, as well as for environmental monitoring and protection and in food industry.

WO2004/086044 discloses the production of nanosensors based on silver nanoparticles. The use of nanosphere lithography is mentioned.

### SUMMARY OF THE INVENTION

The invention in its broadest form is defined by independent claim 1:
A Diagnostic-Nanosensor that is assembled on a spring wire or catheter or stent and that can be used under *in vivo* conditions, comprising:
   a) a deposition of nanopatterned islands of metal on planar or non-planar, transparent or non-transparent surfaces, said nanopatterned islands comprising a nanostructured periodic array of metallic nanoislands assembled of a various morphology and made up of noble metals, wherein the metallic nanoislands are produced by nanosphere lithography,
   b) subsequent deposition of bio-organic interactive material on the deposition described under a) to give a sandwich device for interacting with organic or inorganic targets.
      substances and lead to an increase in sensitivity of the nano-biosensor in terms of lower limit and dynamic range of detention.

It is the primary objective of this invention to address the novel multifunctional biosensor technology based on metallic semiconductors and magnetic nanoislands and nanoisland arrays giving a new nanosensor for diagnostic purposes in medicine, biological and biochemical research, as well as in environmental monitoring and protection and in food industry. The surface of nanoparticles can be functionalized with bio-organic interactive material that due to their specificity interact selectively with other molecules. By chemically controlling the surface of the nanoparticles, they can be "programmed" to recognize and selectively bind molecules, other nanoparticles (semiconductors) or a suitably patterned substrate surface like the cellular outer membrane.
Particularly, it is an objective of this invention to provide nano-transducers based on noble metal nanoislands through resonant excitations of the collective oscillations of the conduction electrons on the nanoislands, the so-called surface plasmon resonances. This phenomenon can be coupled to the binding event between receptors and their targets. Highly improved electrical conductivity of these nano-transducers translate into unprecedented sensitivity and enable the design of novel sensing devices for the detection of pathogens and toxins of concern, to homeland security, food safety, environmental quality, and public health. The use of noble metallic nanoislands results in increased signal transduction of the binding event between bio-organic interactive material and their target substances and lead to an increase in sensitivity of the nano-biosensor in terms of lower limit and dynamic range of detection.

In order to solve the above-defined objects, the present invention provides the Diagnostic Nanosensor according to claim 1.

Preferred embodiments are claimed in the subclaims.

### DESCRIPTION OF THE INVENTION

A highly periodic array of metallic nanoislands acting as nanotranducers forms a new integral structure called a "Diagnostic-Nanosensor". This "Diagnostic-Nanosensor" is deposited on a device that could have a form like a needle or wire or tube or chip and made out of transparent (glass, sapphire) or non-transparent material (silicon substrates, stainless steel, biocompatible polymers). The "Diagnostic-Nanosensor" is made by using nanosphere lithography (NSL) to create metallic nanoislands. This technique allows the total control of size and shape of the functional part of the nanosensor.

One important feature in the optical spectra of nanoislands or nanosized metal particles (Ag, silver; Au, gold) is the surface plasmon band. The surface plasmon band is due to collective electron oscillation around the surface mode of the particles. For example Ag nanoparticles have a strong absorption around 390 nm while Au nanoparticles have the plasmon band around 520 nm. The peak position and bandwidth of the surface plasmon absorption are related to the size and shape. For gold nanorods, however the absorption spectrum changes dramatically and a bimodal spectrum can be detected with peak position dependent on the aspect ratio of the nanorods.

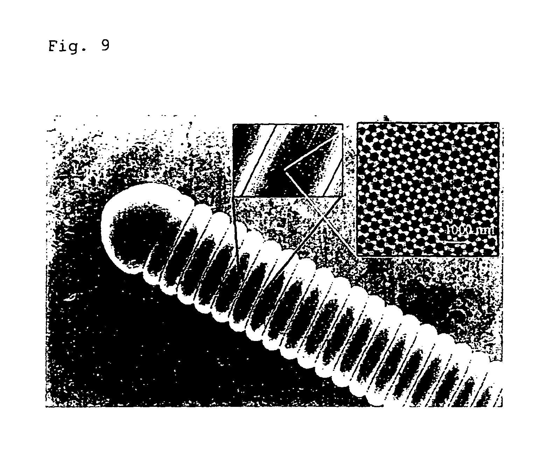

The subsequent modification of nanostructured surfaces of a catheter or spring wire or stent with bio-organic interactive material is presented as an example in Figure 9. This step and especially the binding of DNA, other molecules or cells on the particles surfaces leads to a change in the dielectric constant of this system and can easily be detected by the optical method. The Diagnostic Nanosensor achieves nanometer scale spatial resolution and therefore provides accurate real-time information regarding not only the concentration of a specific analyte of organic or inorganic nature but also its spatial distribution. The operation of sensors made with nanowires, nanotubes, or nano-contacts is based mostly on the reversible change in the optical and electronic properties
In general the sensitivity and specification of the Diagnostic-Nanosensor according to the invention is based on quantum size effect and can be occur with the decreasing the size of our nanoparticles.
The "Diagnostic-Nanosensor" of this invention may be used to detect biological materials, such as proteins, carbohydrates, or nucleic acids or cells as well as non-biological materials, such as synthetic polymers or non organic nanoparticles. The "Diagnostic-Nanosensor" could be used under *in vivo* as well as *in vitro* conditions.

### ADVANTAGES OF THE INVENTION

In the biomedical and human area there is a need for better detection and diagnosis. The nanoscale materials are insatiable for producing new detection and diagnostic devices. On the nanometer length scale, materials exhibit new properties such quantum size confinement. Their surface properties become increasingly important. Nanoparticles made of metals (gold, silver, etc), semiconductors (CdS, CdTe, CdSe, Si, etc) or (superpara-) magnetic particles form an ideal platform for functionalized nanoscale materials. The particles size can be varied from some of nanometer to 100 nm. The small particle size implies high sensitivity and selectivity if they could further functionalized by the precise deposition of bio-organic interactive material for the binding of a given analyte.
The "Diagnostic-Nanosensor" according to the invention is made by using nanosphere lithography (NSL) to create metallic nanoislands. This technique allows the total control of size and shape of the functional part of the nanosensor down to sizes below 10 nm. Subsequent depositions of bio-organic interactive material on the nanostructures give a sandwich device for interacting with organic or inorganic targets. Because the sensitivity and specification of any biosensor depends on the size of a given sensor unit, the invention descript here allows sensitivities far below the pmol range. In general, the Diagnostic-Nanosensor according to the invention is based on quantum size effect and can be tuned with decreasing the size of utilized nanoparticles that are deposited on various support materials and are subsequently functionalized with bio-organic interactive material to give a sandwich device for interacting with organic or inorganic targets.
Assembling the "Diagnostic Nanosensor" on biocompatible supports like spring wire or catheter or stent material as used in medicine the "Diagnostic Nanosensor" gives a molecular/cell select catheter that could be applied for *in vivo* diagnostic purposes. As such the "Diagnostic Nanosensor" according to the invention is for the direct detection and isolation of rare molecules or cells out of the peripheral blood or the body. This application technique enables diagnostic procedures that were not possible before: prenatal diagnoses of chromosomal aberration using foetal trophoblasts present in maternal circulation; cancer diagnoses and monitoring of cancer therapy based on the detection of disseminated cancer cells in the body.
The "Diagnostic Nanosensor" according to the invention allows detection of a resultant interaction between the bio-organic interactive material and the target substance by measuring the collective oscillations of the conduction electrons on the nanoislands, the so called surface plasmon resonance, or detection of optical properties from semiconductors particles (luminescence), or the super-paramagnetic properties by magnetic particles. These detection methods enable the use of the "Diagnostic Nanosensor" according to the invention on scene, i.e. without the infrastructure of a laboratory. Thus, the "Diagnostic Nanosensor" could be used at the bedside, in the operating theatre, in the ambulance, and on battle field.

The surface treatment of various supports with NSL and subsequent functionalizing of the obtained nanostructured array with bio-organic interactive material leads to surprising results

### METHODS FOR PRODUCING THE "DIAGNOSTIC-NANOSENSOR"

The methods for producing the diagnostic-nanosensor comprise two separate but interrelated procedures:
(1) The production of periodic arrays of nanostructures.
(2) Functionalizing nanostructured arrays using bioorganic interactive material that could be bound to the arrays directly or indirectly through linker technologies.

### Ad (1): Production of periodic arrays of nanostructures

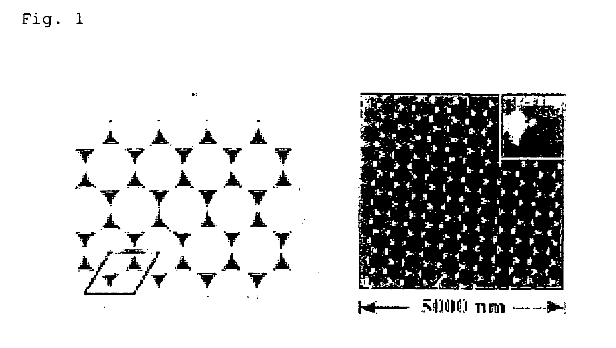

To produce periodic arrays of nanostructures, a relatively inexpensive and simple method of nanoparticles or nanospheres lithography (NSL) will be used for the creation of periodic and quasi-periodic self assembly spherical objects like polystyrene or silica particles with a diameter in the range of 200 to 2000 nm (Figure 1a). The two and three-dimensional (2-D, 3-D) ordered particles will be used as a mask for the following deposition of various amounts of different metals (Au, Ag, Pt) or semiconductors (CdS, CdTe, CdSe). After the evaporation process the liftoff of the mask will be performed. The metallic 2-D (Figure 1b) or 3-D varying particle arrays permit the control of the distance between the nanoparticles as well as their cubic, hexagonal or more complicated ordering. The amount of the deposited materials through the mask can determine directly their optical and electronic properties.

The production of periodic arrays of nanostructures involves the following steps:
a) Production of a two dimensional mask that consists of spherical monodisperse polystyrene particles of sizes from 200 nm to 7000 nm. This production involves the controlled aggregation of the particles between solvents of different surface behaviour for the precise structuring of the two dimensional mask. The final morphology of the mask is determined using temperature and mechanical treatment to give triangles, roots, dots or others.
b) Deposition of the mask on planar substrates (plates or chip) or non-planar substrates (catheters, spring wires, stents). The substrates could be biocompatible for *in vivo* applications of the sensor.
c) Evaporation of noble metals, semiconductors or magnetic materials (gold, platinum, silver, CdSe, CdTe, Cobalt, Nickel) through the mask.
d) Dissolving the mask by temperature or chemical treatment.

### Ad (2): Functionalizing nanostructured arrays

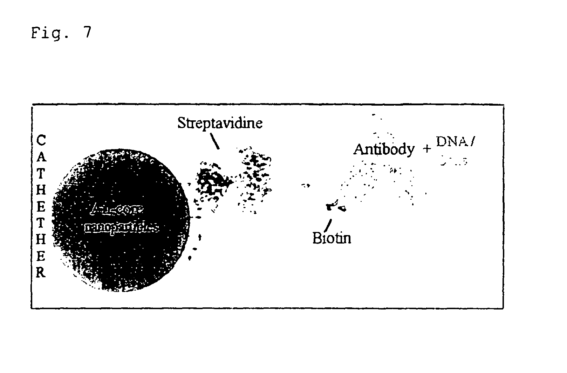

To functionalize nanostructured arrays, bio-organic interactive material is bound to array surfaces using various strategies. These strategies include standard streptavidin-biotin interaction as shown in Figure 7. It also includes the direct binding of (monoclonal) antibodies like IgG or fragments there of to the array surface via adhesion or electrostatic interaction. In addition, covalent linkage of target-specific molecular structures like antibodies or fragments there of, oligomers made of nucleic acids, or peptide or glycopeptides could be performed using linker technology as described in the detailed example below. The latter technique allows the precise binding of target specific molecules to nanoislands giving nanometer scale spatial resolution and therefore provides accurate real-time information regarding not only the concentration of a specific analyte but also its spatial distribution.

### APPLICATIONS OF THE "DIAGNOSTIC-NANOSENSOR"

The "Diagnostic-Nanosensor" is for medical application, for biological and biochemical research, as well as for environmental monitoring and protection and food safety. Concerning medical application, the "Diagnostic-Nanosensor" is assembled as a device for *in vivo* use.

### The "Diagnostic-Nanosensor" for in vivo use

For the *in vivo* device the "Diagnostic Nanosensor" is assembled on spring wire or stents giving a molecular or cell select catheter to obtain rare molecular or cellular components directly out of the circulation or the body in general (Figure 9).

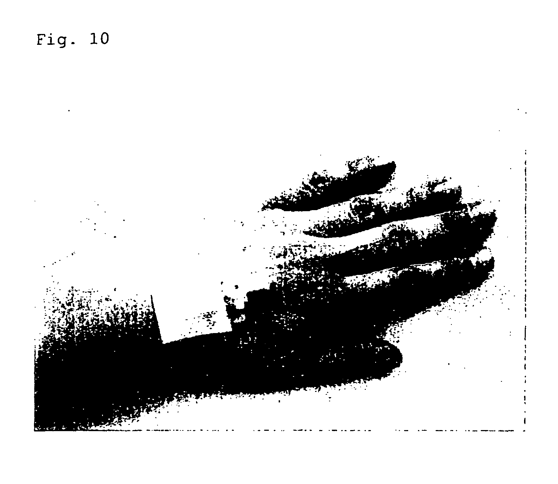

As device for *in vivo* appliance the "Diagnostic-Nanosensor" assembled on e.g. spring wires or stents gives a cell or molecular select catheter which is useful for prenatal diagnosis of chromosomal aberrations, cancer diagnosis and the monitoring of chronic diseases like cancer, metabolic, infectious, allergic and inflammatory diseases. This device is applied as descript below.
a) Puncture of a blood vessel (vein or artery) using a hollow needle.
b) Putting the nanosensor through the hollow needle or through a standard venous line into the blood vessel as shown in Figure 10.
c) Replacing the needle but keeping the nanosensor in place.
d) After the appropriate incubation time (between 5 to 60 minutes) the nanosensor is replaced with the attached cells and/or molecules for further processing.

As a cell select catheter the "Diagnostic Nanosensor" according to the invention is for the isolation of rare cells out of the peripheral blood, e.g. trophoblasts. Trophoblasts are foetal cells that appear in maternal blood around the 7^{th} gestational week at a concentration of about 1-2 per ml. The cell select catheter equipped with a monoclonal antibody that is directed against cellular but not soluble HLA-G binds specifically circulating trophoblasts in maternal blood. The number of bound cells to the cell select catheter depends on the retention period in the maternal circulation and the actual cell concentration. Appropriate numbers of trophoblasts for prenatal diagnoses of chromosomal aberration and other genetic defects could be collected after a retention period of 30±15 minutes *in vivo.* After replacing of the catheter the tip of the catheter with the attached cells is placed into a collector tube for transport to specialized laboratories.

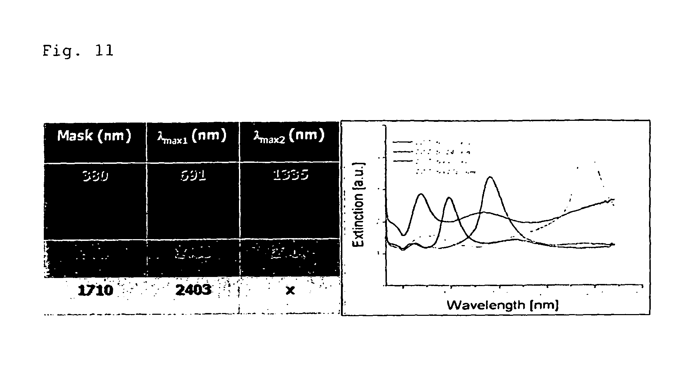

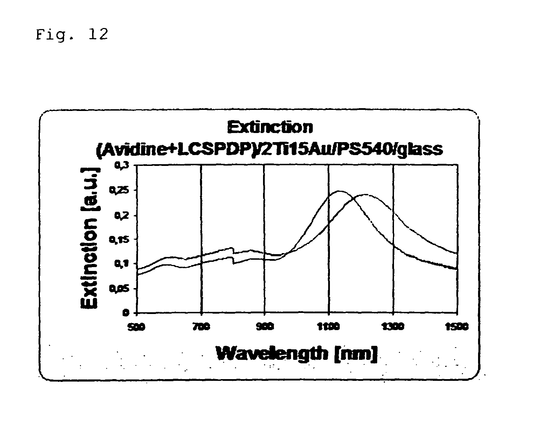

According to the quantum size confinement effect nanosized metallic particles show different optical absorption spectra depending on size. The surface plasmon band is due to collective electron oscillation around the surface of the particles. For example Ag (Silver) nanoparticles have plasmon band around 390 nm while Au nanoparticles have the plasmon band around 520 nm. The pick position, intensity and bandwidth of the surface plasmon absorption are directly related to size, morphology (shape), and surface functionality of the particles (see Figure 11&12).

By covering the 2-dimensional assemblies of gold nanoparticles with avidin their plasmon peak shifts toward longer wavelength and can be easily detected as shown in Fig. 12.

The invention may be further illustrated by the following examples:

### Example 1:

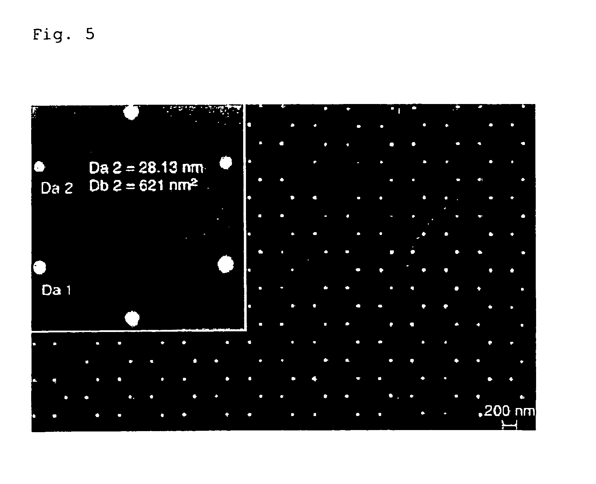

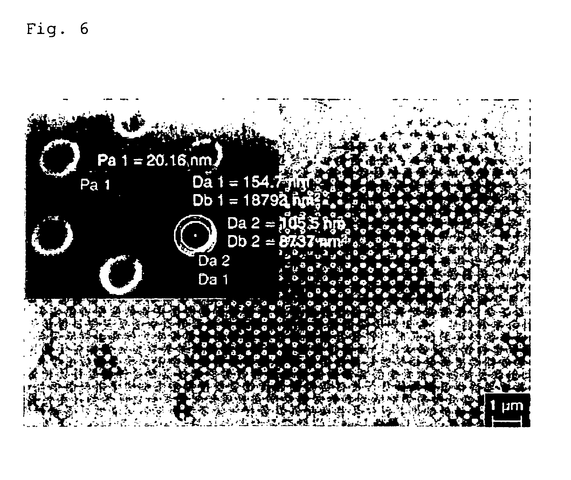

The two-dimensional ordering of latex particles as a basis for nanosphere lithography (NSL) has been used by us and others in the past¹,²,³,⁴,⁵,⁶,⁷,⁸. The evaporation or sputtering of different materials through a latex particle mask enabled the production of lattices of triangular islands on various substrates, the shape of the islands being determined by the shape of the aperture between the spheres in the mask. Clearly, the size of the aperture between the spheres depends on the size of the beads in the monolayer. To overcome this limitation, we annealed the monolayer to downsize the apertures in the mask from an initial 200 nm to 30 nm. This result was far beyond our expectations, and was essential for the fabrication of nanodot particles (Figure 5) and isolated nanorings (Figure 6).
¹H.W. Deckman, J. H. Dunsmir, Appl. Phys. Lett. 1982, 41, 377.
²M. Giersig, P. Mulvaney, Langmuir 1993, 9, 3408.
³ H.W. Deckman, J. H. Dunsmuir, Appl. Phys. Lett. 1982, 4, 377.
⁴ J. C. Hulteen, R. P. Van Duyne, J. Vac. Sci. Technol. A 1995, 13, 1553.
⁵ M. Winzer, M. Kleiber, N. Dix, R. Wiesendanger, Appl. Phys. A 1996, 63, 617.
⁶ R. Micheletto, H. Fukuda, M. Ohtsu, Langmuir 1995, 11, 3333.
⁷F. Burmeister, W. Badowsky, T. Braun, S. Wieprich, J. Boneberg, P. Leiderer, Appl. Surf. Sci. 1999, 461, 144.
⁸ N. D. Denkov, O. D. Velev, P. A. Kralchevsky, I. B. Ivanov, H. Yoshimura, K. Nagayama, Langmuir 1992, 8, 3183.

In our recent work we demonstrated that if the sample is moving during the deposition process, the evaporated structures can become much more complex and range from cuplike structures to rods and wires.⁹ Now, we improved our technique further. Improvements involve decreasing the size of the apertures between particles by temperature treatment applied to the nanosphere mask before the evaporation process, and using different material in nanometer size for the deposition on various supports.⁹
A. Kosiorek, W. Kandulski, P. Chudzinski, K. Kempa, M. Giersig, Nano Lett. 2004, 4, 1359.

### 1. Nanosphere Mask Preparation

A piece of silicon, stainless steal, glass or sapphire substrate (1 cm²) could be used for the deposition of polystyrene (PS) latex particles. In this first step, the latex particles (diameter=540 nm) in ethanol/water solution are slowly applied to the surface of water in a 15 cm Petri dish using a glass pipette. The amount of solution that could be distributed to cover the whole water surface with a hexagonal close packed (hcp) monolayer is limited by the size of the Petri dish and the diameter of the spheres. About 70% of this amount of solution is applied to the surface of the water, which left some places for stress relaxation and avoided the formation of cracks in the lattice during the next steps of the preparation. At this stage the monolayer has the biggest crystals of about 2 cm², with very irregular shapes. To promote the growth of large crystals, gentle waves are applied to the liquid medium by slow and careful vessel tilting. After this treatment, crystals of about 25 cm² are created that show clear diffraction colors. Finally, the monolayer is deposited on a substrate by slow water evaporation.

### 2. Annealing

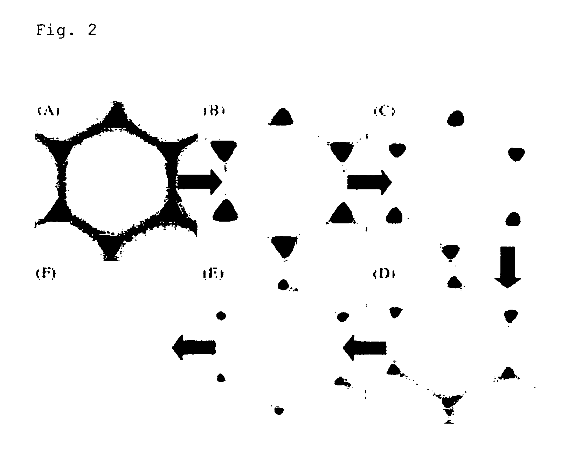

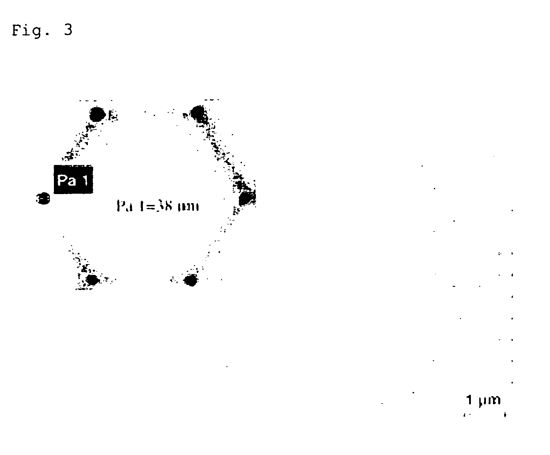

After the drying process, annealing of masks is performed. The monolayer of 540-nm PS latex nanospheres on a conductive Si substrate is immersed in a mixture (25 mL) of water/ethanol/acetone (3:1:1). Microwave heating is used up to the boiling temperature of the mixture, then additional microwave pulses are applied with duration of 3 s and period of 28 s. The increasing number of pulses causes gradual mask annealing and a decrease of the size of the apertures. The dependence of aperture morphology on number of pulses is presented in Figure 2. The liquid environment of the sample ensured very homogeneous annealing-the feature sizes become uniform over the whole area of the sample. The control of the aperture size was very good and reproducible. With the 540-nm PS latex monolayer we were able to adjust the aperture size from an initial 200 nm to 25 nm (Figures 2 and 3). The annealed masks have different thicknesses-the more the sample is annealed, the flatter and thinner it is.

### 3. Metal Deposition

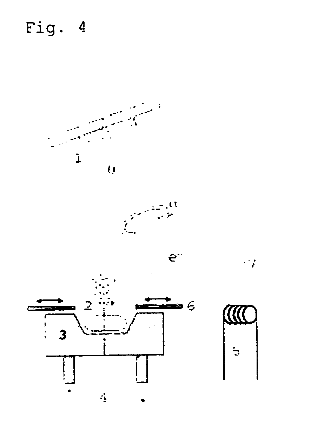

Metal deposition is carried out by evaporation using an e-beam evaporator. The evaporation system is modified to ensure control over the evaporation angle (è) and the sample rotation at the same time (Figure 4).

### 4. Preparation of Nanostructures with Different Morphology

An annealed mask with 30-nm apertures (Figure 3) is used for the fabrication of Co magnetic nanoparticles or Au nanoparticles in a honeycomb lattice. A layer of Co or Au is evaporated (10 nm at 0.01 nms⁻¹) with the sample placed perpendicular to the evaporation beam. The result for Co particles is presented in Figure 5. The size of the Co nanodot particles varies in a small regime and some of them are missing. These differences are all over the sample and arise during the annealing process, most probably because of the small differences (CV=2.4%) in PS sphere diameters.

We used similar masks for nanoring fabrication. In this experiment Fe was deposited (100 nm) through the annealed template from Figure 3 at the evaporation angle θ=258, and the sample was rotated during deposition. The rings had uniform outer (~150 nm) and inner (~100 nm) diameters (inset of Figure 6), which could be controlled by altering the evaporation angle. The thickness of the rings in Figure 6 is about 7 nm and can also be controlled by altering the aperture size of the nanosphere mask and the amount of deposited metal. In a previous paper, we discussed the relationship between the evaporation angle and the amount of deposited metal. Recently there have been many attempts to fabricate nanorings.¹⁰¹¹ However, our method combines a very good control of the dimensions of the structures and fabrication materials with the reproducibility and simplicity of the preparation process.
¹⁰ F. Yan, W. A. Goedel, Nano Lett. 2004, 4, 1193.
¹¹ J. McLellan, M. Geissler, Y. Xia, J. Am. Chem. Soc. 2004, 126, 10830.

### Example 2:

We used the following strategy to specifically bind monoclonal IgG to Au nanoislands that were assembled on transparent or non-transparent substrates.

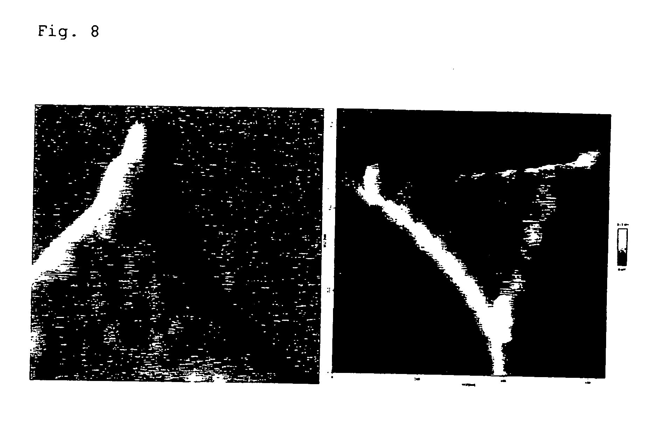

A 20 mM solution of succinimidyl 6-[3'-(2-pyridyldithio)-propionamido] hexanoate, (a SPDP reagent)¹² in DMSO (dimethylsulfoxide) or ethanol was incubated with our periodic arrays of nanostructures in the presence of 150 mM DTT (dithiothreitol) for 30 minutes. This procedure allows covalent linkage of thiol to Au nanoislands.
After cleaning the arrays with buffer (PBS-EDTA) bioorganic interactive material at concentrations from 0.5 to 2 mg/ml was incubated with the array for 1 h or over night. We revealed coatings as shown in Figure 8 for the case that monoclonal IgG at a concentration of 1 mg/ml was used. The dimension of a single dot spot on gold is 12 nm, corresponding to the average molecular weight of a single IgG molecule of 150 kD.
¹² *N*-succinimidyl 3-(2-pyridyldithio) propionate (SPDP reagents) are a unique group of amine- and sulfhydryl-reactive heterobifunctional cross-linkers. Whether they are used to form amine-to-amine or amine-to-sulfhydryl cross-links among molecules, the SPDP reagents produce disulfide-containing linkages that may be cleaved later with reducing agents such as dithiothreitol (DTT). The amine-reactive portion of SPDP reagents is the *N-*hydroxysuccinimide (NHS) ester. Reactions are most commonly performed in phosphate, carbonate/bicarbonate, or borate buffers at pH 7-8. Other buffers may be used provided they do not contain primary amines (or thiols or disulfide reducing reagent). The rate of reaction and degradation by hydrolysis increases with increasing pH; for example, the half-life of the NHS ester is several hours at pH 7 and less than 10 minutes at pH 9. NHS-ester reagents like SPDP and LC-SPDP have limited aqueous solubility and must be dissolved in organic solvent before adding them to a reaction mixture. Sulfo-NHS-ester reagents like Sulfo-LCSPDP are water-soluble and may be added directly to aqueous reaction mixtures.
The sulfhydryl reactive portion of SPDP reagents is the 2-pyridyldithio group, which reacts optimally with sulfhydryls between pH 7 and 8. The reaction results in displacement of a pyridine-2-thione group, the concentration of which may be determined by measuring the absorbance at 343 nm. Reaction buffers must be free of thiols and disulfide reducing agents until quenching or reduction of the 2-pyridyl disulfide is desired. Two basic strategies may be used to form cleavable cross-links between proteins with SPDP reagents, depending whether one or neither protein already possesses sulfhydryl groups (-SH) in addition to primary amines. Both conjugation methods result in cross-links that contain a disulfide bond in the spacer arm, which may be cleaved by reduction with dithiothreitol (DTT) or other reducing agent. In most cases, cross-links created using SPDP reagents can be cleaved with 25 mM DTT at pH 4.5 without reducing native protein disulfide bonds. However, when preservation of native disulfide bonds is not a concern, cleavage with DTT may be performed most efficiently at pH 7-9. Cross-linking experiments with SPDP reagents are not limited to those involving proteins. Any of a variety of molecules with primary amines (-NH2) and sulfhydryl groups may be modified or crosslinked using an SPDP reagent.

### Captions of figures

- Figure 1:: Schematic illustration (a) and typical AFM image (b) of gold nanoparticles after evaporation process and liftoff of the mask
- Figure 2:: A 540-nm PS latex mask annealed in 25 mL of water/EtOH/acetone mixture by A) 1, B) 2, C) 4, D) 6, E) 7, and F) 10 microwave pulses.
- Figure 3:: SEM image of the annealed 540-nm PS latex mask with sizes of all apertures below 50 nm.
- Figure 4:: Diagram of the modified evaporation system: 1) sample holder, 2) evaporation source, 3) crucible, 4) water cooling system, 5) electron beam source, 6) shutter, 7) magnetic field. θ is the evaporation angle and α is the rotation angle of the sample.
- Figure 5:: Ordered spherical Co nanoparticles evaporated through an annealed 540-nm PS latex mask. The diameter of a single particle is approximately 30 nm.
- Figure 6:: SEM picture of ordered Fe nanorings evaporated through an annealed 540-nm PS latex mask. The outer diameter of the single ring is 150 nm and the width of the ring is 20-30 nm.
- Figure 7:: Graphic presentation of how DNA or cellular structures are detected by specific antibodies. Antibodies are cross linked through streptavidin-biotin to Au-nanoparticles. Au-nanoparticles decorate the nanostructured surface of a catheter or spring wire or stent. This technique is the first nano-sandwich approach for the production of a Diagnostic Nanosensor.
- Figure 8:: IgG molecules covalently tethered to Au nanoislands through SPDP linker. The diameter of a single dot is 12 nm, corresponding to the average molecular weight of IgG (150 kD).
- Figure 9:: Diagnostic nanosensors assembled on spring wire to give a cell or molecular select catheter.
- Figure 10:: Cell select catheter in place. A spring wire with diagnostic nanosensors assembled on its surface is put into a peripheral vein through a standard venous line. After appropriate incubation time the cell select catheter is replaced with rare molecules or cells of interest attached to its surface for further laboratory analyses.
- Figure 11:: Influence of particle size on plasmon resonance. Different size of the mask (380, 540, 980, or 1710 nm) gives correspondent nanoparticle depositions on transparent substrate that generate typical spectra.
- Figure 12:: Typical example of spectrum changes of the plasmon resonance after deposition of bio-interactive material (avidin in this case) on nanostructured arrays.

## Claims

1. A Diagnostic-Nanosensor that is assembled on a spring wire or catheter or stent and that can be used under in vivo conditions, comprising:
a) a deposition of nanopatterned islands of metal on planar or non-planar, transparent or non-transparent surfaces, said nanopatterned islands comprising a nanostructured periodic array of metallic nanoislands assembled of a various morphology and made up of noble metals, wherein the metallic nanoislands are produced by nanosphere lithography,
b) subsequent deposition of bio-organic interactive material on the deposition described under a) to give a sandwich device for interacting with organic or inorganic targets.

2. Diagnostic-Nanosensor according to claim 1, wherein said nanostructured periodic array of metallic nanoislands is assembled of nano-needles, wires, tubes, rings or spheres.

3. Diagnostic-Nanosensor according to claim 1 or 2, wherein the said metallic-islands are made up of the noble metals gold, platinum, and silver.

4. The Diagnostic-Nanosensor of one of the preceding claims, wherein the said planar or non-planar substrate is made of stainless steel, glass, silicon or biocompatible polymers and could be in general transparent, non-transparent, conductive, or non-conductive.

5. The Diagnostic-Nanosensor of the preceding claims, wherein the said bio-organic interactive material could be antibodies or part of antibodies, peptides, oligomers made of nucleic acids, or in general receptor structured organic or inorganic compounds that specifically interact with targets.

6. The Diagnostic-Nanosensor according to one of the preceding claims, wherein the nanosphere lithography enable the creation of monodisperse, surface-confined metallic nanotriangles.

7. The Diagnostic-Nanosensor according to one of the preceding claims, wherein the bio-organic interactive material is linked to the nanopatterned structures through adhesion, electrostatic interaction, chemically linkage, or covalent or non-covalent binding.

8. The Diagnostic-Nanosensor according to one of the preceding claims, wherein the binding of target substance to the bioconjugated metallic surface induces a collective interaction with the surface electron (plasmon resonance).

9. The Diagnostic-Nanosensor according to one of the preceding claims, wherein the binding of target substance to the bioconjugated semiconductor surface creates an exiton interaction which could be detected by measuring luminescence or fluorescence.

10. The Diagnostic-Nanosensor according to one of the preceding claims, wherein the binding of target substance to the bioconjugated magnetic surface can be measured by the interaction between ferro- and/or superparamagnetic materials.

11. The Diagnostic-Nanosensor according to one of the preceding claims, wherein every metallic or non-metallic nanoislands act as individual sensor.

## Patentansprüche

1. Diagnostischer Nanosensor, der auf einem Federdraht oder Katheter oder Stent zusammengesetzt ist und der unter In-vivo-Bedingungen verwendet werden kann, umfassend:
(a) eine Aufbringung von nanogemusterten Inseln aus Metall auf planaren oder nichtplanaren, transparenten oder nichttransparenten Oberflächen, wobei die nanogemusterten Inseln eine nanostrukturierte periodische Feldanordnung von metallischen Nanoinseln umfassen, die sich aus einer verschiedenartigen Morphologie zusammensetzen und aus Edelmetallen bestehen, wobei die metallischen Nanoinseln durch Nanosphärenlithografie erzeugt werden,
(b) anschließende Aufbringung eines bioorganischen interaktiven Materials auf der unter (a) beschriebenen Aufbringung, um eine schichtartige Vorrichtung für eine Wechselwirkung mit organischen oder anorganischen Zielen bzw. Targets zu erhalten.

2. Diagnostischer Nanosensor nach Anspruch 1, wobei sich die nanostrukturierte periodische Feldanordnung von metallischen Nanoinseln aus Nanonadeln, Drähten, Rohren, Ringen oder Kugeln bzw. Sphären zusammensetzt.

3. Diagnostischer Nanosensor nach Anspruch 1 oder 2, wobei die metallischen Inseln aus den Edelmetallen Gold, Platin und Silber bestehen.

4. Diagnostischer Nanosensor nach einem der vorhergehenden Ansprüche, wobei das planare oder nichtplanare Substrat aus rostfreiem Stahl, Glas, Silizium oder bioverträglichen Polymeren besteht und im Allgemeinen transparent, nichttransparent, leitfähig oder nichtleitfähig sein kann.

5. Diagnostischer Nanosensor nach einem der vorhergehenden Ansprüche, wobei das bioorganische interaktive Material Antikörper oder ein Teil von Antikörpern, Peptide, aus Nukleinsäuren bestehende Oligomere oder im Allgemeinen rezeptorstrukturierte organische oder anorganische Verbindungen sein kann, die spezifisch mit Zielen bzw. Targets wechselwirken.

6. Diagnostischer Nanosensor nach einem der vorhergehenden Ansprüche, wobei die Nanosphärenlithografie die Erzeugung von monodispersen, oberflächenbeschränkten metallischen Nanodreiecken ermöglicht.

7. Diagnostischer Nanosensor nach einem der vorhergehenden Ansprüche, wobei das bioorganische interaktive Material mit den nanogemusterten Strukturen durch Adhäsion, elektrostatische Wechselwirkung, chemische Verkettung bzw. Bindung oder kovalente oder nichtkovalente Bindung verbunden ist.

8. Diagnostischer Nanosensor nach einem der vorhergehenden Ansprüche, wobei die Bindung einer Zielsubstanz an die biokonjugierte metallische Oberfläche eine kollektive Wechselwirkung mit dem Oberflächenelektron (Plasmonenresonanz) induziert.

9. Diagnostischer Nanosensor nach einem der vorhergehenden Ansprüche, wobei die Bindung einer Zielsubstanz an die biokonjugierte Halbleiteroberfläche eine Exitonenwechselwirkung erzeugt, die durch Messen der Lumineszenz oder Fluoreszenz erfasst werden kann.

10. Diagnostischer Nanosensor nach einem der vorhergehenden Ansprüche, wobei die Bindung einer Zielsubstanz an die biokonjugierte magnetische Oberfläche durch die Wechselwirkung zwischen ferro- und/oder superparamagnetischen Materialien gemessen werden kann.

11. Diagnostischer Nanosensor nach einem der vorhergehenden Ansprüche, wobei jede bzw. alle metallischen oder nichtmetallischen Nanoinseln als individuelle Sensoren wirken.

## Revendications

1. Nano-capteur diagnostique qui est assemblé sur un fil à ressort ou un cathéter ou un stent et qui peut être utilisé dans des conditions in vivo, comprenant :
a) un dépôt d'îlots dotés de nano-motifs en métal sur des surfaces planes ou non planes, transparentes ou non transparentes, lesdits îlots dotés de nano-motifs comprenant un réseau périodique nano-structuré de nano-îlots métalliques assemblés à partir de diverses morphologies et constitués de métaux nobles, les nano-îlots métalliques étant produits par lithographie nanosphères ;
b) un dépôt subséquent de matériau interactif bio-organique sur le dépôt décrit en a) pour former un dispositif en sandwich destiné à interagir avec des cibles organiques ou inorganiques.

2. Nano-capteur diagnostique selon la revendication 1, dans lequel ledit réseau périodique nano-structuré de nano-îlots métalliques est assemblé à partir de nano-aiguilles, fils, tubes, anneaux ou sphères.

3. Nano-capteur diagnostique selon la revendication 1 ou 2, dans lequel lesdits îlots métalliques sont faits des métaux nobles or, platine et argent.

4. Nano-capteur diagnostique selon l'une quelconque des revendications précédentes, dans lequel ledit substrat plan ou non plan est fait d'acier inoxydable, de verre, de silicium ou de polymères biocompatibles, et pourrait être en général transparent, non transparent, conducteur, ou non conducteur.

5. Nano-capteur diagnostique selon l'une quelconque des revendications précédentes, dans lequel ledit matériau interactif bio-organique pourrait être des anticorps ou une partie d'anticorps, des peptides, des oligomères constitués d'acides nucléiques, ou en général des composés organiques ou inorganiques structurés en récepteurs qui interagissent spécifiquement avec ces cibles.

6. Nano-capteur diagnostique selon l'une quelconque des revendications précédentes, dans lequel la lithographie nanosphères permet la création de nanotriangles métalliques confinés en surface et monodispersés.

7. Nano-capteur diagnostique selon l'une quelconque des revendications précédentes, dans lequel le matériau interactif bio-organique est lié aux structures dotées de nano-motifs par l'intermédiaire d'une adhésion, d'une interaction électrostatique, d'une liaison chimique, ou d'une liaison covalente ou non covalente.

8. Nano-capteur diagnostique selon l'une quelconque des revendications précédentes, dans lequel la liaison de la substance cible à la surface métallique bio-conjuguée induit une interaction collective avec les électrons de surface (résonance plasmonique).

9. Nano-capteur diagnostique selon l'une quelconque des revendications précédentes, dans lequel la liaison de la substance cible à la surface semi-conductrice bio-conjuguée crée une interaction d'exitons qui pourrait être détectée par mesure de luminescence ou de fluorescence.

10. Nano-capteur diagnostique selon l'une quelconque des revendications précédentes, dans lequel la liaison de la substance cible à la surface magnétique bio-conjuguée peut être mesurée par l'interaction entre des matériaux ferro- et/ou superparamagnétiques.

11. Nano-capteur diagnostique selon l'une quelconque des revendications précédentes, dans lequel chacun des nano-îlots métalliques ou non métalliques agit comme un capteur individuel.
